# EUROPEAN PATENT SPECIFICATION

(11) **EP 0 424 667 B1**
(45) Date of publication and mention of the grant of the patent: **14.12.1994**
(21) Application number: 90118072.9
(22) Date of filing: 20.09.1990
(51) Int. Cl.: A61L 2/08

(54) **A salt restored from discoloration and a method for this restoration**
Salz, dessen ursprüngliche Farbe nach einer Verfärbung wiederhergestellt wurde, sowie ein Verfahren für diese Wiederherstellung
Sel qui était décoloré et dont on a restauré la couleur naturelle, procédé pour cette restauration

(30) Priority: 24.10.1989 SE 8903513
(43) Date of publication of application: 02.05.1991
(73) Proprietor: GAMBRO AB, S-220 10 Lund (SE)
(72) Inventor: Lindqvist, Sten-Börje, S-240 14 Veberöd (SE)
(74) Representative: Asketorp, Göran

(56) References cited:
- No relevant documents have been disclosed.

## Description

### TECHNICAL FIELD

The present invention relates to a salt sterilized by radioactive radiation e g gamma irradiation and/or beta irradiation of the type which obtains a discoloration on irradiation. In the first place this refers to salts intended to be used for medical purposes, e g in dialysis for the preparation of dialysis liquid.

A probable explanation of the discoloration is that it is due to an electron displacement, that is to say to one or more electrons being made to change electron orbit and thereby getting into an excited state.

### BACKGROUND ART

Unpublished experiments aimed at restoring the original colour of the irradiated salt have been carried out with the help of daylight, UV-ligt and microwaves. However, no evident effect was achieved.

Alternatively it is possible to refrain from irradiation and use instead a bacteria filter for the filtering out of undesirable substances after the salt has been dissolved in water or other suitable liquid. Difficulties can arise here in maintaining this bacteria filter sterile. They may be overcome, though, in the manner which is described, for example, in the American patent specification 4 783 273.

A further method consists in using a powder which is as pure as possible, and dissolving the same directly in connection with its utilization. See, for example, American patent specification 4 784 495. It is not possible here, however, to guarantee absolute sterility.

### DISCLOSURE OF INVENTION

The salt in accordance with the invention, that is to say a salt sterilized by radio-active radiation, e g gamma irradiation and/or beta irradiation of the type which obtains a discoloration on irradiation is characterized in that its original colour is wholly or partly restored by heat during a time period depending on the temperature and long enough to restore the original colour at least partly or recrystallization in a wholly sterile environment. In this manner the problems mentioned above can be fully solved.

In particular the invention relates in the first place to sodium hydrogen carbonate and sodium chloride, that is to say salts which are frequently used in connection with, for example, dialysis, hemofiltration and plasmaferes. The salts may be dissolved directly in conjunction with the medical treatment, as described in the above mentioned American patent 4 784 495. Also by such a use it is, however, desirable for the powder to be present in the form of a wholly sterile natural-coloured powder.

The invention also related to the actual method for restoring the orginal colour of a salt sterilized by radioactive radiation of the type which obtains a discoloration on irradiation. This method is characterized in that it takes place with the help of heating during a time period depending on the temperature and long enough to restore the original colour at least partly.

Insofar as the treatment of irradiated sodium hydrogen carbonate is concerned, the temperature and duration of treatment may be selected according to approximately the following relations:
a) one day at 65°C resulting in a diminishing discoloration,
b) three days at 75°C resulting in a natural-coloured substantially white powder,
c) one day at 85°C resulting in a natural-coloured, substantially white powder,
d) 6 hours at 95°C resulting in a natural-coloured, substantially white powder,
e) 3 hours at 105°C resulting in a natural-coloured, substantially white powder.
For information it may be added that one day's treatment at approximately 55°C did not produce any objectively detectable change of colour.

Furthermore, it has been found that no changes of temperatures or durations of treatment were required when changing the irradiation dose for sodium hydrogen carbonate from 25kGy to 50 kGy. On the other hand it has been established that sodium hydrogen carbonate of analytically pure quality obtains a less pink coloration than the corresponding standard quality for medical usage.

Insofar as the treatment of irradiated sodium chloride is concerned, the temperature and duration of treatment should be selected according to the following relation:
one day at 150°C resulting in a natural-coloured, substantially white powder.

For information it may be added that a treatment at 100°C during one day proved to have no effect on the powder coloured orange-brown as a result of irradiation.

Alternatively the discoloration can be eliminated by means of recrystallization with the help of sterile water. In the process a saturated solution is produced from which the salt can be precipitated again, thereafter to be separated from surplus liquid and dried. The whole has to be carried out, of course, in completely sterile environment and the same applies to subsequent storage which may be done, for example, in small completely bacteria-tight packages.

Naturally the invention is not limited solely to the examples described above but can be varied within the framework of the subsequent claims. For example it will be clear to those versed in the art that there are probably common properties of many salts which bring about the discoloration obtained on radioactive irradiation. For this reason is should be possible for many salts other than those mentioned as examples to be treated by the method in accordance with the invention.

## Claims

1. A salt sterilized by radioactive radiation, e g gamma irradiation and/or beta irradiation, of the type which obtains a discoloration on irradiation, **characterized** in that its original colour is wholly or partly restored by means of heat or recrystallization in a wholly sterile environment.

2. A salt in accordance with claim 1, **characterized** in that it consists of sodium hydrogen carbonate or sodium chloride.

3. A salt in accordance with claim 1 or 2, **characterized** in that its original colour is restored by means of heat during a time period depending on the temperature and long enough to restore the original colour at least partly.

4. A method for restoring the original colour of a salt sterilized by radioactive radiation of the type which obtains a discoloration on irradiation, **characterized** in that it is carried out with the help of heating during a time period depending on the temperature and long enough to restore the original colour at least partly.

5. A method in accordance with claim 4 in the treatment of irradiated sodium hydrogen carbonate **characterized** in that temperature and duration of treatment are selected according to the following relations:
a) one day at 65°C resulting in a diminishing discoloration,
b) three days at 75°C resulting in a natural-coloured substantially white powder,
c) one day at 85°C resulting in a natural-coloured substantially white powder,
d) six hours at 95°C resulting in a natural-coloured, substantially white powder,
e) three hours at 105°C resulting in a natural-coloured, substantially white powder.

6. A method in accordance with claim 4 or 5, **characterized** in that duration and temperature are selected independently of the dose of radiation which may be, for example, 25 kGy or 50 kGy.

7. A method in accordance with claim 4 in the treatment of irradiated sodium chloride, **characterized** in that temperature and duration of treatment are selected according to the following relation:
one day at 150°C resulting in a natural-coloured, substantially white powder.

8. A method for restoring the original colour of a salt sterilized by radioactive radiation of the type which obtains a discoloration on irradiation, **characterized** in that it takes place with the help of recrystallization in a wholly sterile environment.

9. A method in accordance with claim 8, **characterized** in that the irradiation salt is dissolved with the help of sterile water, made into a saturated solution, caused to precipitate, separated from surplus water and dried.

## Patentansprüche

1. Durch radioaktive Strahlung, z. B. gamma-Bestrahlung und/oder beta-Bestrahlung, sterilisiertes Salz des Typs, welcher sich bei Bestrahlung verfärbt, **dadurch gekennzeichnet**, daß seine ursprüngliche Farbe durch Wärme oder Umkristalisation in einer völlig sterilen Umgebung ganz oder teilweise wiederhergestellt wird.

2. Salz nach Anspruch 1, **dadurch gekennzeichnet**, daß es aus Natriumhydrogencarbonat der Natriumchlorid besteht.

3. Salz nach Anspruch 1 oder 2, **dadurch gekennzeichnet**, daß seine ursprüngliche Farbe durch Wärme während einer Zeitdauer wiederhergestellt wird, die von der Temperatur abhängt und lange genug ist, um die ursprüngliche Farbe wenigstens teilweise wiederherzustellen.

4. Verfahren zur Wiederherstellung der ursprünglichen Farbe eines durch radioaktive Strahlung sterilisierten Salze des Typs, der sich bei Bestrahlung verfärbt, **dadurch gekennzeichnet,** daß es mit Hilfe von Erhitzen während einer Zeitdauer, die von der Temperatur abhängt und lange genug ist, um die ursprüngliche Farbe wenigstens teilweise wiederherzustellen, durchgeführt wird.

5. Verfahren nach Anspruch 4 bei der Behandlung von bestrahltem Natriumhydrogencarbonat, **dadurch gekennzeichnet**, daß die Behandlungstemperatur und -dauer gemäß den folgenden Beziehungen ausgewählt werden:
a) ein Tag bei 65 °C, was zu einer Verfärbungverminderung führt,
b) drei Tage bei 75 °C, was zu einem natürlich gefärbten, im wesentlichen weißen Pulver führt,
c) ein Tag bei 85 °C, was zu einem natürlich gefärbten, im wesentlichen weißen Pulver führt,
d) 6 h bei 95 °C, was zu einem natürlich gefärbten, im wesentlichen weißen Pulver führt,
e) 3 h bei 105 °C, was zu einem natürlich gefärbten, im wesentlichen weißen Pulver führt.

6. Verfahren nach Anspruch 4 oder 5, **dadurch gekennzeichnet**, daß die Dauer und Temperatur unabhängig von der Strahlungsdosis ausgewählt werden, die beispielsweise 25 kGy oder 50 kGy sein kann.

7. Verfahren nach Anspruch 4 bei der Behandlung von bestrahltem Natriumchlorid, **dadurch gekennzeichnet**, daß die Behandlungstemperatur und -dauer gemäß der folgenden Beziehung ausgewählt werden:
ein Tag bei 150 °C, was zu einem natürlich gefärbten, im wesentlichen weißen Pulver führt.

8. Verfahren zur Wiederherstellung der ursprünglichen Farbe eines durch radioaktive Strahlung sterilisierten Salzes des Typs, welcher sich bei Bestrahlung verfärbt, **dadurch gekennzeichnet**, daß es mit Hilfe von Umkristallisation in einer völlig sterilen Umgebung stattfindet.

9. Verfahren nach Anspruch 8, **dadurch gekennzeichnet**, daß das Bestrahlungssalz mit Hilfe von sterilem Wasser aufgelöst, in eine gesättigte Lösung überführt, zur Ausfällung gebracht, von überschüssigem Wasser getrennt und getrocknet wird.

## Revendications

1. Sel stérilisé par un rayonnement radiocatif, par exemple une irradiation gamma et/ou une irradiation bêta du type qui aboutit à une décoloration par irradiation, caractérisé en ce que sa couleur d'origine est entièrement ou partiellement restituée au moyen de la chaleur ou d'une recristallisation dans un environnement totalement stérile.

2. Sel selon la revendication 1, caractérisé en ce qu'il est constitué d'hydrogénocarbonate de sodium ou de chlorure de sodium.

3. Sel selon la revendication 1 ou 2, caractérisé en ce que sa couleur d'origine est restituée au moyen de la chaleur pendant une période de temps dépendant de la température et suffisamment longue pour restituer la couleur d'origine au moins partiellement.

4. Procédé pour restituer la couleur d'origine d'un sel stérilisé par un rayonnement radioactif du type qui aboutit à une décoloration par irradiation, caractérisé en ce qu'il est effectué à l'aide de la chaleur pendant une période de temps dépendant de la température et suffisamment longue pour restituer la couleur d'origine au moins partiellement.

5. Procédé selon la revendication 4 dans le traitement de l'hydrogénocarbonate de sodium irradié, caractérisé en ce que la température et la durée du traitement sont choisies dans les rapports suivants :
a) un jour à 65°C aboutit à une décoloration décroissante,
b) trois jours à 75°C aboutissent à une poudre de couleur naturelle sensiblement blanche,
c) un jour à 85°C aboutit à une poudre de couleur naturelle sensiblement blanche,
d) 6 heures à 95°C aboutissent à une poudre de couleur naturelle sensiblement blanche,
e) 3 heures à 105°C aboutissent à une poudre de couleur naturelle sensiblement blanche.

6. Procédé selon la revendication 4 ou 5, caractérisé en ce que la durée et la température sont choisies indépendamment du taux de rayonnement qui peut être, par exemple, de 25 kGy ou de 50 kGy.

7. Procédé selon la revendication 4, dans le traitement du chlorure de sodium irradié, caractérisé en ce que la température et la durée du traitement sont choisies dans le rapport suivant : un jour à 150°C aboutit à une poudre de couleur naturelle sensiblement blanche.

8. Procédé pour restituer la couleur d'origine d'un sel stérilisé par un rayonnement radioactif du type qui aboutit à une décoloration par irradiation, caractérisé en ce qu'il est effectué à l'aide d'une recristallisation dans un environnement totalement stérile.

9. Procédé selon la revendication 8, caractérisé en ce que le sel d'irradiation est dissous à l'aide d'eau stérile, mis sous forme d'une solution saturée, amené à précipiter, séparé de l'excès d'eau et séché.
